# FASCICULE DE BREVET EUROPEEN

(11) **EP 3 166 581 B1**
(45) Date de publication et mention de la délivrance du brevet: **17.02.2021**
(21) Numéro de dépôt: 15748282.9
(22) Date de dépôt: 09.07.2015
(51) Int. Cl.: A61K 8/34, A61K 8/60, A61Q 13/00, A61Q 15/00, A61K 8/73, A61K 8/11, A61K 8/25, A61K 8/26, A61K 8/29, A61K 8/19

(54) **COMPOSITION ANHYDRE A BASE DE POUDRE COIFFANTE ET/OU ABSORBANTE DE SEBUM ET DE PARTICULES ENCAPSULANT UN AGENT BÉNÉFIQUE**
WASSERFREIE ZUBEREITUNG AUF BASIS EINES HAARSTYLING-PULVERS UND/ODER EINES SEBUM-ABSORBIERENDEN PULVERS UND PARTIKELN ENTHALTEND VERKAPSELTER PFLEGESTOFF
ANHYDROUS COMPOSITION BASED ON HAIRSTYLING POWDER AND/OR SEBUM-ABSORBING POWDER AND PARTICLES ENCAPSULATING A BENEFIT AGENT

(30) Priorité: 09.07.2014 FR 1456629; 09.07.2014 FR 1456632; 29.10.2014 FR 1460404
(43) Date de publication de la demande: 17.05.2017
(73) Titulaire: L'OREAL, 75008 Paris (FR)
(72) Inventeur: AUBERT, Lionel, F-95270 Asnieres sur Oise (FR); BEAU, Nathalie, F-95610 Eragny-sur-Oise (FR)
(74) Mandataire: L'Oreal
(86) Numéro de dépôt international: PCT/FR2015/051896
(87) Numéro de publication internationale: WO 2016/005703

(56) Documents cités:
- EP-A1- 0 974 332
- EP-A1- 1 407 754
- DE-A1-102008 035 013
- FR-A1- 2 990 133
- US-A1- 2014 079 747
- "Refresh Dry Shampoo", GNPD; MINTEL, avril 2010 (2010-04), XP002690820, [extrait le 2010-04-01]

## Description

Dans le domaine du lavage des matières kératiniques, les shampooings secs existent depuis de nombreuses années, soit sous une forme poudreuse, soit sous forme d'aérosol. Ils permettent d'éliminer l'excès de sébum d'une façon rapide sans mouiller la chevelure. Ils agissent en captant le sébum par absorption grâce à des poudres choisies pour leurs qualités absorbantes de sebum (décrits par exemple dans les documents FR2 990 133 A1 et "Refresh Dry Shampoo", GNPD ID 1317319; MINTEL, avril 2010).

Les poudres employées peuvent être d'origine minérale, organique ou synthétique et peuvent être des dérivés d'amidon de blé, de riz et de maïs.

Dans la pratique, les shampooings secs proposés ne donnent pas entière satisfaction. En particulier, la sensation de propreté perçue par les utilisateurs n'est pas persistante tout au long de la journée, notamment après des activités physiques.

Il existe donc un besoin de mettre au point une nouvelle composition d'un shampooing sec qui offre à la fois une activité nettoyante optimale et un apport de volume à la chevelure, avec une sensation de propreté durable dans le temps.

La demanderesse a trouvé de manière étonnante et avantageuse que l'utilisation d'au moins une poudre absorbante de sébum et/ou d'une poudre coiffante en association avec des particules encapsulant au moins un agent bénéfique pouvant libérer l'agent bénéfique en présence d'eau permettait d'offrir les propriétés nettoyantes attendues d'un shampooing sec ainsi que des propriétés coiffantes telles qu'un apport de volume et de masse à la chevelure avec une sensation de propreté, de fraîcheur persistante dans le temps.

La composition selon l'invention permet notamment la libération contrôlée de l'agent bénéfique en fonction de la présence d'eau/humidité au niveau du cuir chevelu et/ou des cheveux, en particulier suite à des activités physiques.

Par ailleurs, la composition selon l'invention est stable, en particulier les capsules d'agent bénéfique ne sont pas dégradées au sein de la composition.

Lorsque la composition se présente sous forme d'aérosol, la composition peut être délivrée de façon satisfaisante.

Par ailleurs, la composition de l'invention laisse moins de résidus blancs que les produits classiquement utilisés.

Enfin, la composition de l'invention présente une bonne tolérance au niveau du cuir chevelu.

L'invention a donc pour objet une composition anhydre comprenant :
a) au moins des particules encapsulant au moins un agent bénéfique, les particules étant aptes à libérer l'agent bénéfique en présence d'eau;
b) au moins une poudre absorbante de sébum présentant une prise de sébum supérieure ou égale à 35 ml/100 g et/ou au moins une poudre coiffante différente(s) des particules encapsulant au moins un agent bénéfique ;
c) un agent propulseur, caractérisée en ce que les particules encapsulant au moins un agent bénéfique comprennent au moins un matériau choisi parmi les amidons naturels ou modifiés ; et que l'agent bénéfique encapsulé est une substance parfumante.

Par «composition anhydre», on entend au sens de la présente invention, une composition présentant une teneur en eau inférieure à 5 % en poids, de préférence inférieure à 2% en poids, et/ou une composition qui ne contient pas d'eau ajoutée, c'est-à-dire que l'eau éventuellement présente dans la composition selon l'invention est plus particulièrement de l'eau liée, comme l'eau de cristallisation des sels ou des traces d'eau absorbées par les matières premières utilisées dans la réalisation des compositions selon l'invention.

Selon l'invention, la composition peut comprendre une ou plusieurs **poudres absorbantes de sébum** présentant une prise de sébum supérieure ou égale à 35 ml/100 g.

Par poudre absorbante de sébum, on entend au sens de la présente invention une poudre apte à absorber et/ou adsorber le sébum qui présente une prise de sébum supérieure ou égale à 35 ml/100 g.

La prise de sébum correspond à la quantité de sébum absorbé et/ou adsorbé par la poudre. Elle est exprimée en ml de sébum pour 100 g. de poudre et mesurée à l'aide de la méthode de détermination de prise d'huile de poudre décrite dans la norme NF T 30-022.

La prise d'huile de poudre correspond à la quantité de sébum absorbé sur la surface disponible de la poudre par mesure du 'Wet point' comme indiqué ci-dessous.

La méthode de mesure est la suivante : on place une quantité m (en grammes) comprise entre 0,5 et 5 grammes de poudre sur une plaque de verre, la quantité dépendant de la densité de la poudre, puis on ajoute goutte à goutte du sébum artificiel ayant la composition suivante :

| | |
|---|---|
| - Trioléine | 29 % en poids |
| - Acide oléique | 28,5 % en poids |
| - Oléate d'oléyle | 18,5 % en poids |
| - Squalène | 14 % en poids |
| - Cholestérol | 7 % en poids |
| - Palmitate de cholestérol | 3 % enpoids |

Après addition de 4 à 5 gouttes de sébum artificiel, on incorpore le sébum artificiel dans la poudre à l'aide d'une spatule et on continue d'ajouter du sébum artificiel jusqu'à la formation de conglomérats de sébum artificiel et de poudre. A partir de ce moment, on ajoute le sébum artificiel à raison d'une goutte à la fois, et on triture ensuite le mélange avec la spatule.

On cesse l'addition de sébum artificiel lorsque l'on obtient une pâte ferme et lisse. Cette pâte doit se laisser étendre sur la plaque de verre sans craquelures ni formation de grumeaux. On note alors le volume Vs en ml, de sébum artificiel utilisé.

La prise de sébum correspond au rapport Vs/m.

La ou les poudre(s) absorbante(s) de sébum utilisée(s) dans le dispositif aérosol de l'invention présente(nt) une prise de sébum de préférence, allant de 35 à 1000 ml/100 g, mieux encore de 35 à 800 ml/100 g.

Avantageusement, la particule absorbant le sébum peut avoir une surface spécifique BET supérieure ou égale à 150 m2/g, de préférence supérieure ou égale à 300 m2/g, mieux supérieure à 500 m2/g, et préférentiellement supérieure à 600 m2/g, et notamment inférieure à 1500 m2/g.

La « surface spécifique BET » est déterminée selon la méthode BET (BRUNAUER - EMMET - TELLER) décrite dans « The journal of the American Chemical Society », vol. 60, page 309, février 1938 et correspondant à la norme internationale ISO 5794/1 (annexe D). La surface spécifique BET correspond à la surface spécifique totale (donc micropores compris) de la particule, et notamment de la poudre.

La poudre absorbante de sébum peut être une poudre minérale ou une poudre organique.

Plus précisément, la poudre absorbante de sébum peut être choisie parmi :
les amidons,
les silicates de calcium,
les perlites,
les zéolithes,
les acides polylactiques,
les silices,
les poudres de polyamides (nylon®),
les poudres de polymères acryliques, notamment de polyméthacrylate de méthyle, de poly(méthacrylate de méthyle/diméthacrylate d'éthylène glycol), de poly(méthacrylate d'allyle/diméthacrylate d'éthylène glycol), de copolymère diméthacrylate d'éthylène glycol/méthacrylate de lauryle ;
les poudres de silicone élastomère, notamment obtenues par polymérisation d'organopolysiloxane ayant au moins deux atomes d'hydrogène liés chacun à un atome de silicium et d'un organopolysiloxane comprenant au moins deux groupes à insaturation éthylénique (notamment deux groupes vinyles) en présence de catalyseur platine ; et
leurs mélanges.

La poudre absorbante de sébum peut être une poudre enrobée avec un agent de traitement hydrophobe.

L'agent de traitement hydrophobe peut être choisi parmi les acides gras comme l'acide stéarique ; les savons métalliques comme le dimyristate d'aluminium, le sel d'aluminium du glutamate de suif hydrogéné ; les acides aminés ; les acides aminés N-acylés ou leurs sels ; la lécithine, le trisostéaryle titanate d'isopropyle, et leurs mélanges.

Les acides aminés N-acylés peuvent comprendre un groupe acyle ayant de 8 à 22 atomes de carbones, comme par exemple un groupe 2-éthyl hexanoyle, caproyle, lauroyle, myristoyle, palmitoyle, stéaroyle, cocoyle. Les sels de ces composés peuvent être les sels d'aluminium, de magnésium, de calcium, de zirconium, de zinc, de sodium, de potassium. L'acide aminé peut être par exemple la lysine, l'acide glutamique, l'alanine.

Le terme alkyl mentionné dans les composés cités précédemment désigne notamment un groupe alkyle ayant de 1 à 30 atomes de carbone, de préférence ayant de 5 à 16 atomes de carbone.

Les amidons utilisables dans la présente invention sont, par exemple, l'amidon de maïs, l'amidon de pomme de terre, l'amidon de tapioca, l'amidon de riz, l'amidon de blé, et l'amidon de cassaya.

Les amidons peuvent être modifiés ou non.

Un amidon modifié est un amidon qui a été modifié par des procédés connus de l'homme de l'art, comme par exemple l'estérification, l'éthérification, l'oxydation, l'hydrolyse acide, la réticulation, ou la conversion enzymatique.

Des exemples non limitatifs d'amidon modifié comprennent l'octénylsuccinate-amidon d'aluminium, l'octénylsuccinate-amidon de sodium, l'octénylsuccinate-amidon de calcium, le phosphate de diamidon, l'hydroxyéthyl-amidon de phosphate, l'hydroxypropyl-amidon de phosphate, le carboxyméthyl-amidon de sodium, et le glycolate-amidon de sodium.

Dans un mode de réalisation particulier, l'amidon est un octénylsuccinate d'amidon, en particulier d'aluminium, l'amidon étant de maïs, de blé ou de riz. On peut citer notamment le produit proposé par AKZO NOBEL sous la dénomination DRY FLO PLUS.

De préférence, les silicates de calcium utilisés comme poudre absorbante de sébum présente une prise de sébum supérieure à 200 ml/100 g, mieux entre 400 ml/100 g et 600 ml/100 g, plus préférentiellement d'environ 475 ml/100 g.

L'aire de surface spécifique (BET) va de préférence d'environ 150 m2/g à 600 m2/g, mieux de 300 m2/g à 600 m2/g, et encore plus préférentiellement de 310 m2/g à 350 m2/g.

La taille des particules des silicates est de préférence inférieure à 20 micromètres.

Ces silicates de calcium sont généralement préparés par réaction de silice réactive avec un réactif de métal alcalino-terreux, de préférence un oxyde ou hydroxyde de métal alcalino-terreux, et une source d'aluminium tels que l'aluminate de sodium ou de l'alumine. Les propriétés finales du silicate étant fonctions de la réactivité de la silice, la source de silice préférée est le produit de réaction d'un silicate soluble, tel que du silicate de sodium, et d'un acide minéral tel que l'acide sulfurique. Des silicates de métaux alcalino-terreux synthétiques amorphes appropriés sont fabriqués par la société JM Huber Corporation et sont vendus sous les dénominations Hubersorb®. Des méthodes de préparation de ces silices sont divulguées plus en détail dans le brevet US 4 557 916. D'autres silicates appropriés sont disponibles chez JM Huber Corporation comme l'aluminosilicate de sodium vendu sous la marque Zeolexg et l'aluminosilicate de magnésium et de sodium vendu sous la marque Hydrex®.

On peut également utiliser comme poudre absorbante de sébum, les perlites qui sont généralement des aluminosilicates d'origine volcanique et qui ont pour composition :
70,0-75,0% en poids de silice SiO2
12,0-15,0% en poids d'oxyde d'aluminium oxyde Al2O3
3,0-5,0% d'oxyde de sodium Na2O
3,0-5,0% d'oxyde de potassium K2O
0,5-2% d'oxyde de fer Fe2O3
0,2-0,7% d'oxyde de magnésium MgO
0,5-1,5% d'oxyde de calcium CaO
0,05 - 0,15% d'oxyde de titane TiO2.

A titre d'exemples de zéolithes, on peut notamment citer les composés d'aluminosilicates de sodium ou de potassium tels que le produit proposé par ZEOCHEM sous l'appellation XMOL.

Les acides polylactiques utilisables dans la présente invention sont notamment l'ACCUREL EP600 d'AKZONOBEL ou le produit proposé sous la dénomination LACTIC ACID POLYMER 9105 par DAJAC LABS.

Comme poudre de silice, on peut citer :
- les microsphères de silice poreuses vendues sous la dénomination SILICA BEADS SB-700 par la société MYOSHI ; "SUNSPHERE® H51", "SUNSPHEREO H33" par la société ASAHI GLASS ;
- les microsphères de silice amorphe enrobées de polydiméthylsiloxane vendues sous la dénomination "SA SUNSPHERE® H 33", "SA SUNSPHERE® H53" par la société ASAHI GLASS.

Comme poudre de nylon, on peut citer la poudre de nylon vendue sous la dénomination ORGASOL® 4000 par la société ATOCHEM.

Comme poudre de polymères acryliques, on peut citer :
- les poudres de polyméthacrylate de méthyle vendus sous la dénomination COVABEAD® LH85 par la société WACKHERR ;
- les poudres de poly méthacrylate de méthyle/diméthacrylate d'éthylène glycol vendues sous la dénomination DOW CORNING 5640 MICROSPONGE® SKIN OIL ADSORBER par la société DOW CORNING ; GANZPEARL® GMP-0820 par la société GANZ CHEMICAL ;
- les poudres de polyméthacrylate d'allyle/diméthacrylate d'éthylène glycol vendues sous la dénomination POLY-PORE® L200, POLY-PORE® E200 par la société AMCOL Health and Beauty Solutions Inc. ; ces dernières ont notamment une prise de sébum supérieure ou égale à 1 ml/g, mieux allant de 1 ml/g à 20 ml/g ;
- les poudres de copolymère diméthacrylate d'éthylène glycol/méthacrylate de lauryle vendues sous la dénomination POLYTRAP® 6603 de la société DOW CORNING.

Comme poudre de silicone élastomère, on peut citer les poudres vendues sous les dénominations "Trefil® Powder E-505C", "Trefil® Powder E-506C" par la société DOW CORNING.

De préférence, la poudre absorbante de sébum est choisie parmi les amidons modifiés tels que les octénylsuccinates d'amidon, et en particulier d'aluminium, la perlite, les acides polylactiques, et les zéolithes, mieux parmi les octénylsuccinates d'amidons.

La ou les poudre(s) absorbante(s) de sébum peut (peuvent) être présente(s) en une quantité allant de préférence de 0,1 à 99 % en poids, mieux encore de 1 à 90 % en poids, et encore plus préférentiellement de 2 à 80 % en poids par rapport au poids total de la composition.

La composition peut également comprendre une ou plusieurs **poudres coiffantes** différente(s) des poudres absorbantes de sébum.

Par « poudre coiffante » on entend une poudre constituée de un ou plusieurs composé(s) minéral(aux) insoluble(s) dans l'eau présentant une aptitude à la mise en forme de la chevelure ou à la durabilité de cette mise en forme.

Le(s) composé(s) minéral(aux) insoluble(s) dans l'eau sont choisis parmi les carbonates, oxydes et sulfates métalliques et les silicates contenant du magnésium.

Par insoluble dans l'eau, on entend au sens de la présente invention un composé dont la solubilité à pH spontané dans l'eau à 25°C et à pression atmosphérique est inférieure à 0,1%.

A titre d'exemples, on peut plus particulièrement citer les carbonates, oxydes et sulfates de métaux alcalino-terreux comme le béryllium, le magnésium, le calcium, le strontium, le baryum et le radium, mieux le magnésium et le calcium ; les oxydes, sulfates et carbonates d'aluminium, de gallium et d'indium ; et les silicates contenant du magnésium, en particulier ceux contenant une quantité de magnésium supérieure à 10% en poids (en base sèche) exprimée en oxyde de magnésium tels que les silicates de Li-Mg-Na comme la Laponite XLG proposée par la société ROCKWOOD.

Plus préférentiellement, on utilisera le carbonate de calcium, le carbonate de magnésium, l'alumine, le sulfate de baryum, et/ou l'oxyde de magnésium, et mieux encore le carbonate de calcium. De préférence, ces composés présentent une taille moyenne de particules de 20 à 50 µm, comme composé(s) minéral(aux) insoluble(s) dans l'eau.

Le(s) composé(s) minéral(aux) insoluble(s) dans l'eau peut(peuvent) être présent(s) en une quantité allant de 0,1 à 99% en poids, mieux encore de 1 à 90% en poids, et encore plus préférentiellement de 2 à 80 % en poids par rapport au poids total de la composition

Selon l'invention, la composition comprend des **particules encapsulant au moins un agent bénéfique,** les particules étant aptes à libérer l'agent bénéfique en présence d'eau.

Par «agent bénéfique», on entend au sens de l'invention tout composé présent dans un produit de consommation produisant un effet bénéfique perçu par le consommateur lors de son utilisation et/ou obtenu sur le produit de consommation lui-même, ledit effet bénéfique pouvant être une amélioration sensorielle ou une modification notamment visuelle et/ou olfactive et/ou tactile, une amélioration du confort et/ou de la facilité d'application, un effet esthétique, un effet hygiénique, une sensation de propreté, un effet curatif et/ou prophylactique.

Les particules encapsulant au moins un agent bénéfique utilisées dans la composition selon l'invention sont aptes à libérer l'agent bénéfique en présence d'eau.

Au moins un agent bénéfique est immobilisé, capturé et/ou encapsulé dans la matrice d'un système d'encapsulation ou de piégeage; ledit agent bénéfique étant libéré vers l'extérieur au fur et à mesure de la détérioration du système d'encapsulation ou de piégeage lorsque sa dégradation se produit au contact d'eau.

Les particules encapsulant au moins un agent bénéfique utilisées dans la composition selon l'invention peuvent comprendre un cœur contenant au moins un agent bénéfique et une enveloppe entourant le cœur. Ces particules sont creuses (vésiculaires) et on parle de microcapsules.

Par «particules comprenant un cœur contenant au moins un agent bénéfique», on entend une particule comprenant au moins un agent bénéfique immobilisé, capturé et/ou encapsulé dans la matrice d'un système d'encapsulation ou de piégeage; ledit agent bénéfique étant libéré vers l'extérieur au fur et à mesure de la détérioration du système d'encapsulation ou de piégeage lorsque sa dégradation se produit au contact d'un milieu avec lequel il va réagir ou sous l'effet d'un stimulus tel qu'un apport d'eau.

Alternativement, les particules encapsulant au moins un agent bénéfique utilisées dans la composition selon l'invention peuvent comprendre une matrice poreuse, l'agent bénéfique étant contenu dans les pores de la matrice. Ces particules sont pleines (matricielles) et on parle de microsphères.

Les particules encapsulant au moins un agent bénéfique utilisées dans la composition selon l'invention comprennent les amidons naturels ou modifiés.

Selon un mode de réalisation préféré, les particules encapsulant au moins un agent bénéfique comprennent un cœur et une enveloppe comprenant au moins un matériau choisi parmi les amidons naturels ou modifiés.

Les particules encapsulant au moins un agent bénéfique utilisées dans la composition selon l'invention peuvent être sphériques. Par «sphériques», on entend que la particule présente un indice de sphéricité, c'est-à-dire le rapport entre son plus grand diamètre et son plus petit diamètre, est inférieur à 1,2. Dans ce cas, de telles particules sont généralement appelées «capsules».

Les particules sphériques conformes à la présente invention présentent, de préférence, un diamètre moyen en nombre allant de 1 à 30 µm plus préférentiellement allant de 2 à 15 µm et encore mieux de 5 à 10 µm et un diamètre moyen en volume allant de 5 à 150 µm, de préférence allant de 10 à 100 µm et encore mieux de 20 à 80 µm.

Par « diamètre moyen » des particules on entend les paramètres D[4,3] ou D[1,0] mesurés en voie sèche par diffraction laser, par exemple à l'aide d'un granulomètre Microtrac S3500, les résultats étant exprimés sous la forme de distributions granulométriques en volume ou en nombre respectivement donnant accès au diamètre moyen.

**L'agent bénéfique** encapsulé est une substance parfumante.

Par «substance parfumante», on entend tout ingrédient susceptible de dégager une odeur agréable.

Les parfums sont des compositions contenant notamment des matières premières (dénommées généralement ingrédients de parfumerie) qui sont décrites dans S. Arctander, Perfume and Flavor Chemicals (Montclair, N.J., 1969), dans S. Arctander, Perfume and Flavor Materials of Natural Origin (Elizabeth, N.J., 1960) et dans "Flavor and Fragrance Materials - 1991", Allured Publishing Co. Wheaton, III.

Il peut s'agir de produits de synthèse ou de produits naturels, comme des huiles essentielles, des absolus, des résinoïdes, des résines, des concrètes, et/ou des produits synthétiques (hydrocarbures terpéniques ou sesquiterpéniques, alcools, phénols, aldéhydes, cétones, éthers, acides, esters, nitriles, peroxydes, saturés ou insaturés, aliphatiques ou cycliques).

Selon la définition donnée dans la norme internationale ISO 9235 et adoptée par la Commission de la Pharmacopée Européenne, une huile essentielle est un produit odorant généralement de composition complexe, obtenu à partir d'une matière première végétale botaniquement définie, soit par entraînement à la vapeur d'eau, soit par distillation sèche, soit par un procédé mécanique approprié sans chauffage. L'huile essentielle est le plus souvent séparée de la phase aqueuse par un procédé physique n'entraînant pas de changement significatif de la composition.

Parmi les huiles essentielles utilisables selon l'invention, on peut citer celles obtenues à partir des plantes appartenant aux familles botaniques suivantes :
Abiétaceés ou Pinacées : conifères; Amaryllidacées; Anacardiacées; Anonacées : ylang; Apiacées (par exemple les ombellifères) : aneth, angélique, coriandre, criste marine, carotte, persil; Aracées; Aristolochiacées; Astéracées : achilée, armoise, camomille, hélichryse; Bétulacées; Brassicacées; Burséracées : encen ; Caryophyllacées; Canellacées; Césalpiniacées : copaïfera (copahu); Chénopodacées; Cistacées : ciste; Cypéracées; Diptérocarpacées; Ericacées : gaulthérie (wintergreen); Euphorbiacées; Fabacées; Geraniacées : géranium; Guttifères; Hamamélidacées; Hernandiacées; Hypéricacées : millepertuis; Iridacées; Juglandacées; Lamiacées : thym, origan, monarde, sarriette, basilic, marjolaines, menthes, patchouli, lavandes, sauges, cataire, romarin, hysope, mélisse; Lauracées : ravensara, laurier, bois de rose, cannelle, litséa; Liliacées : ail, lys, muguet, jacinthe, jonquille,...; Magnoliacées : magnolia; Malvacées ; Méliacées; Monimiacées; Moracées : chanvre, houblon; Myricacées; Mysristicacées : muscade; Myrtacées : eucalyptus, tea tree, niaouli, cajeput, backousia, girofle, myrte; Oléacées; Pipéracées : poivre ; Pittosporacées; Poacées : citronnelle, lemongrass, vétiver; Polygonacées; Renonculacées; Rosacées : roses; Rubiacées; Rutacées : tous les citrus; Salicacées; Santalacées : santal; Saxifragacées; Schisandracées; Styracacées : benjoin; Thymélacées : bois d'agar; Tilliacées; Valérianacées : valériane, nard; Verbénacées : lantana, verveine; Violacées; Zingibéracées : galanga, curcuma, cardamome, gingembre; Zygophyllacées.

On peut citer également les huiles essentielles extraites de fleurs (lys, lavande, rose, jasmin, Ylang-Ylang, néroli), de tiges et de feuilles (patchouli, géranium, petit-grain), de fruits (framboise, pêche ,coriandre, anis, cumin, genièvre), d'écorces de fruits (bergamote, citron, orange, pamplemousse), de racines (angélique, céleri, cardamome, iris, acore, gingembre), de bois (bois de pin, santal, gaïac, cèdre rose, camphre), d'herbes et de graminées (estragon, romarin, basilic, lemon grass, sauge, thym), d'aiguilles et de branches (épicéa, sapin, pin, pin nain), de résines et de baumes (galbanum, élémi, benjoin, myrrhe, oliban, opopanax).

Des exemples de substances parfumantes sont notamment : le géraniol, l'acétate de géranyle, le farnésol, le bornéol, l'acétate de bornyle, le linalool, l'acétate de linalyle, le propionate de linalyle, le butyrate de linalyle, le tétrahydrolinalool, le citronellol, l'acétate de citronellyle, le formiate de citronellyle, le propionate de citronellyle, le dihydromyrcénol, l'acétate de dihydromyrcényle, le tétrahydromyrcénol, le terpinéol, l'acétate de terpinyle, le nopol, l'acétate de nopyle, le nérol, l'acétate de néryle, le 2-phényléthanol, l'acétate de 2-phényléthyle, l'alcool benzylique, l'acétate de benzyle, le salicylate de benzyle, l'acétate de styrallyle, le benzoate de benzyle, le salicylate d'amyle, le diméthylbenzyl-carbinol, l'acétate de trichlorométhylphénylcarbinyle, l'acétate de p-tert-butylcyclohexyle, l'acétate d'isononyle, l'acétate de cis-3-hexenyle, l'acétate de vétivéryle, l'acétate d'éthyle, l'acétate de butyle, l'acétate d'hexyle, l'acétate de décyle, l'acétate d'isoamyle, l'acétate de stéaryle, l'heptanoate d'allyle, le vétivérol, l'alpha-hexylcinnamaldéhyde, le 2-méthyl-3-(p-tert-butylphényl)propanal, le 2-méthyl-3-(p-isopropylphényl)propanal, le 3-(p-tert-butylphényl)-propanal, le 2,4-diméthylcyclohex-3-enyl-carboxaldéhyde, l'acétate de tricyclodécènyle, le propionate de tricyclodécènyle, l'allyl 3-cyclohexylpropionate, l' éthyl-6-(acétyloxy)-hexanoate, le caproate d'allyle, l' éthyl-2 méthyl butyrate, le méthyl dihydrojasmonate, le salicylate d'hexyle, le 4-(4-hydroxy-4-méthylpentyl)-3-cyclohexènecarboxaldéhyde, le 4-(4-méthyl-3-pentènyl)-3-cyclohexènecarboxaldéhyde, le 4-acétoxy-3-pentyl-tétrahydropyrane, le 3-carboxyméthyl-2-pentylcyclopentane, la 2-n-4-heptylcyclopentanone, la 3-méthyl-2-pentyl-2-cyclopentènone, la menthone, la carvone, la tagétone, la géranyl acétone, le n-décanal, le n-dodécanal, l'anisyl propanal, le 9-décènol-1, le cis-3-hexénol, le tétrahydro-2-isobutyl-4-méthylpyrann-4-ol, 3-Methyl-5-phényl-1-pentanol, le 3a,6,6,9a-Tétraméthyl-dodécahydronaphtho[2,1-b]furanne, l'isobutyrate de phénoxyéthyle, le phénylacétaldéhyde diméthyl-acétal, le phénylacétaldéhyde diéthylacétal, le géranonitrile, le citronellonitrile, l'acétate de cédryle, le 3-isocamphylcyclohexanol, le cédryl méthyl éther, l'isolongifolanone, l'aubépinonitrile, l'aubépine, l'héliotropine, la coumarine, l'eugénol, la vanilline, l'oxyde de diphényle, le citral, le citronellal, l'hydroxycitronellal, l'hexylcinnamal, le 2,4-dimethylcyclohex-3-ène-1-carbaldéhyde, le 2,6-dimethylhept-5-ènal, l'α,α-diméthyl-p-éthylphénylpropanal, le 1,3-benzodioxole-5-carboxaldéhyde, le limonène, la damascone, la décalactone, la nonalactone, le 6,6-diméthoxy-2,5,5-triméthylhex-2-ène, la 2,4,4,7-tétraméthyloct-6-èn-3-one, Ia1-(5,5-diméthyl-1-cyclohexenyl)-pent-4-èn-1-one, la méthylheptènone, le 4-(cyclopropylméthyl)-phénylméthyl éther, le 2-methyl-6-methylideneoct-7-en-2-ol, l'oxyde de rose, la 1-(1,2,3,4,5,6,7,8-octahydro-2,3,8,8-tetraméethyl-2-naphthyl)éthan-1-one, la 2-acétonaphthone, la 2-isopropyl-5-méthylcyclohexanone, les ionones, les méthylionones, les isométhylionones, la solanone, les irones, le cis-3-hexénol et ses esters, les muscs-indanes, les muscs-tétralines, les muscs-isochromanes, les cétones macrocycliques, les muscs-macrolactones, les muscs aliphatiques, le brassylate d'éthylène, l'essence de rose et leurs mélanges.

Les particules selon l'invention contenant l'agent bénéfique représentent, de préférence, de 0,01 à 10% en poids, de préférence de 0,05 à 5% en poids et encore mieux de 0,1 à 3% en poids du poids total de la composition.

Les particules encapsulant au moins un agent bénéfique utilisées dans la composition selon l'invention peuvent être obtenues par des techniques d'encapsulation conventionnelles. En particulier, elles peuvent être obtenues selon le procédé décrit dans le brevet US5508259. On réalise notamment une émulsion à partir des amidons naturels ou modifiés, en combinaison avec un agent émulsionnant et un mélange d'ingrédients de parfumerie. L'émulsion est ensuite déshydratée par un procédé classique d'atomisation (spray-drying), qui consiste, tel que décrit à l'exemple 1 du brevet US5508259, à la pulvériser en fines gouttelettes dans un atomiseur avec un débit de 50kg/h et une pression de 0,45 bars, au contact d'un courant d'air de 320m3/h chauffé à 350°C afin d'évaporer l'eau, ce qui permet d'obtenir une poudre fine avec un diamètre de particules compris entre 20 et 80 microns et contenant 20% en poids de parfum.

A titre de particules encapsulant au moins un agent bénéfique on peut citer les capsules APPLE BLOSSOM CAPS 01 commercialisées par la société Firmenich, les capsules EMERALD FIRCAPS commercialisées par la société Firmenich, les capsules TECNI MIX PLUS commercialisées par la société Mane, les capsules INCAP 50 Green Boost, INCAP 50 Clean et INCAP 50 Care commercialisées par la société Symrise.

Comme indiqué précédemment, la composition selon l'invention comprend un ou plusieurs agents propulseurs.

L'agent propulseur est choisi parmi l'air, l'azote, le gaz carbonique, le diméthyléther, les hydrocarbures volatils, tels que notamment les alcanes en C₃₋₅, les hydrocarbures chlorés et/ou fluorés tels que le 1,1-difluoroéthane et leurs mélanges, de préférence choisis parmi les alcanes en C₃₋₅ et de préférence le n-butane, le propane, l'isobutane et leurs mélanges.

Préférentiellement, on peut citer les alcanes en C₃₋₅ et en particulier le propane, le n-butane, l'isobutane et leurs mélanges.

L'agent ou les agents propulseur(s) est ou sont présent(s) en une quantité allant de 10 à 95 % en poids, mieux encore de 15 à 90 % en poids, et encore plus préférentiellement de 20 à 88 % par rapport au poids total de la composition.

La composition de l'invention peut en outre comprendre un ou plusieurs **corps gras liquides.**

Par « corps gras », on entend un composé organique insoluble dans l'eau à température ordinaire (25 °C) et à pression atmosphérique (760 mm de Hg, soit 1,013.105 Pa), c'est-à-dire de solubilité inférieure à 5 % et de préférence inférieure à 1 %, encore plus préférentiellement inférieure à 0,1 %.

De préférence, les corps gras sont non siliciés, c'est-à-dire que leur structure ne comporte pas d'atome de silicium. Ils présentent généralement dans leur structure une chaîne hydrocarbonée comportant au moins 6 atomes de carbone et ne comprenant pas de groupe siloxane.

En outre, les corps gras sont généralement solubles dans des solvants organiques dans les mêmes conditions de température et de pression, comme par exemple le chloroforme, l'éthanol, le benzène, l'huile de vaseline ou le décaméthyl cyclopentasiloxane.

Les corps gras liquides utilisables dans l'invention sont liquides à température ambiante (25 °C) et à pression atmosphérique (760 mm de Hg, soit 1,013.105 Pa). Ils présentent de préférence une viscosité inférieure ou égale à 2 Pa.s, mieux inférieure ou égale à 1 Pa.s et encore mieux inférieure ou égale à 0,1 Pa.s à la température de 25 °C et à un taux de cisaillement de 1 s 1.

Les corps gras liquides utilisables dans la composition selon l'invention ne sont généralement pas oxyalkylénés et de préférence ne contiennent pas de fonction acide carboxylique COOH.

De préférence, les corps gras liquides sont choisis parmi les hydrocarbures, les alcools gras, les esters gras, les éthers gras, et leurs mélanges.

Encore plus préférentiellement, ils sont choisis parmi les hydrocarbures, les alcools gras, les esters gras et leurs mélanges.

Par hydrocarbure liquide, on entend un hydrocarbure composé uniquement d'atomes de carbone et d'hydrogène, liquide à température ordinaire (25 °C) et à pression atmosphérique (760 mm de Hg, soit 1,013.105 Pa), d'origine minérale ou végétale ou synthétique.

Plus particulièrement, les hydrocarbures liquides sont choisis parmi :
- les alcanes en C6-C16 linéaires ou ramifiés, éventuellement cycliques. A titre d'exemples, on peut citer l'hexane, l'undécane, le dodécane, le tridécane et les isoparaffines comme l'isohexadécane, l'isododécane et l'isodécane.
- les hydrocarbures linéaires ou ramifiés, d'origine minérale animale ou synthétique, de plus de 16 atomes de carbone, tels que les huiles de paraffine ou de vaseline, l'huile de vaseline, les polydécènes, le polyisobutène hydrogéné tel que celui vendu sous la marque Parléam® par la société NOF Corporation, le squalane.

Dans une variante préférée, le ou les hydrocarbures liquides sont choisis parmi les huiles de paraffine ou de vaseline.

Par alcool gras liquide, on entend un alcool gras non glycérolé et non oxyalkyléné, liquide à température ordinaire (25 °C) et à pression atmosphérique (760 mm de Hg, soit 1,013.105 Pa).

De préférence, les alcools gras liquides de l'invention comportent de 8 à 30 atomes de carbone.

Les alcools gras liquides de l'invention peuvent être saturés ou insaturés.

Les alcools gras liquides saturés sont de préférence ramifiés. Ils peuvent éventuellement comprendre dans leur structure au moins un cycle aromatique ou non. De préférence, ils sont acycliques.

Plus particulièrement, les alcools gras saturés liquides de l'invention sont choisis parmi l'octyldodécanol, l'alcool isostéarylique, le 2-hexyldécanol.

L'octyldodécanol est tout particulièrement préféré.

Les alcools gras insaturés liquides présentent dans leur structure au moins une double ou triple liaison, et de préférence, une ou plusieurs doubles liaisons. Lorsque plusieurs doubles liaisons sont présentes, elles sont de préférence au nombre de 2 ou 3 et elles peuvent être ou non conjuguées.

Ces alcools gras insaturés peuvent être linéaires ou ramifiés.

Ils peuvent éventuellement comprendre dans leur structure au moins un cycle aromatique ou non. De préférence, ils sont acycliques.

Plus particulièrement, les alcools gras insaturés liquides de l'invention sont choisis parmi l'alcool oléique (ou oléylique), l'alcool linoléique (ou linoléylique), l'alcool linolénique (ou linolénylique), et l'alcool undécylénique.

L'alcool oléique est tout particulièrement préféré.

Par esters gras liquide, on entend un ester issu d'un acide gras et/ou d'un alcool gras, liquide à température ordinaire (25 °C) et à pression atmosphérique (760 mm de Hg, soit 1,013.105 Pa).

Les esters sont de préférence les esters liquides de mono- ou polyacides aliphatiques saturés ou insaturés, linéaires ou ramifiés, en C1-C26 et de mono- ou polyalcools aliphatiques saturés ou insaturés, linéaires ou ramifiés, en C1-C26, le nombre total d'atomes de carbone des esters étant supérieur ou égal à 10.

De préférence, pour les esters de monoalcools, l'un au moins de l'alcool ou de l'acide dont sont issus les esters de l'invention est ramifié.

Parmi les monoesters de monoacides et de monoalcools, on peut citer les palmitates d'éthyle et d'isopropyle, les myristates d'alkyle tels que le myristate d'isopropyle ou d'éthyle, le stéarate d'isocétyle, l'isononanoate de 2-éthylhexyle, l'isononanoate d'isononyle, le néopentanoate d'isodécyle, et le néopentanoate d'isostéaryle.

On peut également utiliser les esters d'acides di- ou tricarboxyliques en C4-C22 et d'alcools en C1-C22 et les esters d'acides mono-, di- ou tricarboxyliques et d'alcools non-sucres di-, tri-, tétra- ou pentahydroxylés en C4-C26.

On peut notamment citer le sébacate de diéthyle, le sébacate de diisopropyle, le sébacate de di(2-éthylhexyle), l'adipate de diisopropyle, l'adipate de di n-propyle, l'adipate de dioctyle, l'adipate de di(2-éthyhexyle), l'adipate de diisostéaryle, le maléate de di(2-éthylhexyle), le citrate de triisopropyle, le citrate de triisocétyle, le citrate de trisostéaryle, le trilactate de glycéryle, le trioctanoate de glycéryle, le citrate de trioctyldodécyle, le citrate de trioléyle, le diheptanoate de néopentyl glycol, et le diisononate de diéthylène glycol.

La composition peut également comprendre, à titre d'ester gras liquide, des esters et di-esters de sucres et d'acides gras en C6-C30, de préférence en C12-C22. Il est rappelé que l'on entend par « sucre », des composés hydrocarbonés oxygénés qui possèdent plusieurs fonctions alcools, avec ou sans fonction aldéhyde ou cétone, et qui comportent au moins 4 atomes de carbone. Ces sucres peuvent être des monosaccharides, des oligosaccharides ou des polysaccharides.

Comme sucres convenables, on peut citer par exemple le saccharose, le glucose, le galactose, le ribose, le fucose, le maltose, le fructose, le mannose, l'arabinose, le xylose, le lactose, et leurs dérivés notamment alkylés, tels que les dérivés méthylés comme le méthylglucose.

Les esters de sucres et d'acides gras peuvent être choisis notamment dans le groupe comprenant les esters ou mélanges d'esters de sucres décrits auparavant et d'acides gras en C6-C30, de préférence en C12-C22, linéaires ou ramifiés, saturés ou insaturés. S'ils sont insaturés, ces composés peuvent comprendre une à trois doubles liaisons carbone-carbone, conjuguées ou non.

Les esters selon cette variante peuvent être également choisis parmi les mono-, di-, tri- et tétraesters, les polyesters et leurs mélanges.

Ces esters peuvent être par exemple des oléates, laurates, palmitates, myristates, béhénates, cocoates, stéarates, linoléates, linolénates, caprates, arachidonates, et leurs mélanges comme notamment les esters mixtes oléo-palmitate, oléo-stéarate, palmitostéarate.

Plus particulièrement, on utilise les mono- et diesters et notamment les mono- ou dioléates, stéarates, béhénates, oléopalmitates, linoléates, linolénates, oléostéarates, de saccharose, de glucose ou de méthylglucose.

On peut citer à titre d'exemple, le produit vendu sous la dénomination Glucate® DO par la société AMERCHOL, qui est un dioléate de méthylglucose.

Enfin, on peut aussi utiliser les esters naturels ou synthétiques de mono-, di- ou triacides avec le glycérol.

Parmi ceux-ci, on peut citer les huiles végétales.

Comme huiles d'origine végétale ou triglycérides synthétiques, utilisables dans la composition de l'invention à titre d'esters gras liquides, on peut citer par exemple :
- les huiles triglycérides d'origine végétale ou synthétique, telles que les triglycérides liquides d'acides gras comportant de 6 à 30 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, d'olive, de colza, de coprah, de germe de blé, d'amande douce, d'abricot, de carthame, de noix de bancoulier, de camélina, de tamanu, de babassu et de pracaxi les triglycérides des acides caprylique/caprique comme ceux vendus par la société STEARINERIES DUBOIS ou ceux vendus sous les dénominations Miglyol® 810, 812 et 818 par la société DYNAMIT NOBEL, l'huile de jojoba, l'huile de beurre de karité.

De préférence, on utilisera à titre d'esters selon l'invention des esters gras liquides issus de monoalcools.

Les myristate ou palmitate d'isopropyle sont particulièrement préférés.

Les éthers gras liquides sont choisis parmi les dialkyléthers liquides tels que le dicaprylyléther.

De préférence, les corps gras liquides sont choisis parmi les hydrocarbures linéaires ou ramifiés d'origine minérale, animale ou synthétique de plus de 16 atomes de carbone, les alcools gras ramifiés ou insaturés, les esters gras, les huiles triglycérides d'origine végétale.

Plus préférentiellement, les corps gras liquides sont choisis parmi les huiles de paraffine ou de vaseline, l'octyldodécanol, l'alcool isostéarylique, le 2-hexyldécanol, l'alcool oléique (ou oléylique), l'alcool linoléique (ou linoléylique), l'alcool linolénique (ou linolénylique), et l'alcool undécylénique, les palmitates d'éthyle et d'isopropyle, les myristates d'alkyle tels que le myristate d'isopropyle ou d'éthyle, le stéarate d'isocétyle, l'isononanoate de 2-éthylhexyle, l'isononanoate d'isononyle, le néopentanoate d'isodécyle, et le néopentanoate d'isostéaryle, les huiles végétales.

Encore plus préférentiellement, les corps gras liquides sont choisis parmi le palmitate d'isopropyle, le myristate d'isopropyle.

Lorsqu'ils sont présents, le(s) corps gras liquides est ou sont présents en une quantité allant de préférence par exemple de 0,05 à 20 % en poids, et mieux de 0,1 à 10% en poids, encore mieux de 0,2 à 5%, en poids par rapport au poids total de la composition.

La composition selon l'invention peut en outre contenir un ou plusieurs additifs choisis parmi les polymères anioniques, cationiques, non ioniques amphotères ou zwittérioniques, conditionneurs ou fixants, les parfums, les colorants, les filtres protecteurs, les acides, les bases, les nacres, les paillettes.

Ces additifs peuvent être présents dans la composition selon l'invention en une quantité allant de 0 à 20 % en poids par rapport au poids total de la composition.

L'homme de métier veillera à choisir ces éventuels additifs et leurs quantités de manière à ce qu'ils ne nuisent pas aux propriétés des compositions de la présente invention.

Les compositions sous forme de poudre conformes à l'invention sont conditionnées dans un dispositif aérosol constitué par un récipient comprenant la composition et par un moyen de distribution de ladite composition.

Le récipient est pressurisé. Le récipient peut être opaque ou transparent. Il peut être en verre, en matériau polymérique ou en métal, recouvert éventuellement d'une couche de vernis protecteur.

Le récipient peut alors renfermer à la fois le ou les agents propulseurs et les autres ingrédients de la composition, en un seul compartiment, ou en variante dans deux compartiments. Selon cette dernière variante, le récipient peut être constitué par un bidon aérosol externe comportant une poche interne soudée hermétiquement à une valve. Les différents ingrédients de la composition sont introduits dans la poche interne et un agent propulseur est introduit entre la poche et le bidon à une pression suffisante pour faire sortir le produit sous forme d'un spray.

Le récipient est équipé à son extrémité supérieure d'une valve assurant l'étanchéité du système.

On pourra par exemple utiliser une valve commercialisée par les sociétés PRECISION, COSTER, SEAQUIST et LINDAL.

Le dispositif, conditionné avec une telle valve, permet d'assurer l'étanchéité du système, ainsi que la distribution du produit depuis le récipient.

La présente invention concerne également un procédé de lavage à sec et de traitement cosmétique des cheveux, comprenant l'application sur les cheveux, de préférence secs, d'une composition telle que décrite ci-dessus.

La présente invention concerne aussi l'utilisation de la composition définie ci-dessus pour le lavage à sec et le traitement cosmétique des cheveux.

### EXEMPLE

Dans l'exemple suivant, toutes les quantités sont indiquées en pourcentage pondéral de produit en l'état par rapport au poids total de la composition.

Les compositions selon l'invention suivantes ont été préparées à partir des composés indiqués dans les tableaux ci-dessous.

| | A |
|---|---|
| DRY FLO PLUS par National Starch (Aluminium Starch Octenylsuccinate à 86%) | 92,15 |
| Carbonate de calcium (D50 = 35µm)¹ | 1,45 |
| Hectorite modifiée avec le chlorure de distéaryldiméthylammonium2 | 2,00 |
| Myristate d'isopropyle | 3,00 |
| Capsules de parfum à base d'amidon³ | 1,40 |

| | |
|---|---|
| ¹ : Vendu sous la dénomination commerciale OMYACARE S60 par Omya ² : Vendu sous la dénomination commerciale Bentone 38 par Elementis ³: Vendu sous la dénomination commerciale APPLE BLOSSOM CAPS par Firmenich. | |

On a introduit cette composition A dans un flacon de type salière. Cet exemple ne fait pas partie de l'invention.

| | B |
|---|---|
| DRY FLO PLUS par National Starch (Aluminium Starch Octenylsuccinate à 86%) | 10,92 |
| Carbonate de calcium (D50 = 35µm)¹ | 2,18 |
| Hectorite modifiée avec le chlorure de distéaryldiméthylammonium² | 0,28 |
| Myristate d'isopropyle | 0,42 |
| Capsules de parfum à base d'amidon³ | 0,19 |
| Isobutane | 86,00 |

| | |
|---|---|
| ¹ : Vendu sous la dénomination commerciale OMYACARE S60 par Omya ² : Vendu sous la dénomination commerciale Bentone 38 par Elementis ³: Vendu sous la dénomination commerciale APPLE BLOSSOM CAPS par Firmenich. | |

On a introduit cette composition B dans un dispositif aérosol. Ledit dispositif est équipé d'une valve classiquement utilisée pour les aérosols. On a appliqué la composition A et la composition B sur une chevelure grasse et sale. L'application est bien tolérée par le cuir chevelu. On observe une sensation de fraîcheur à l'application.

Après séchage on constate que la chevelure est visuellement plus propre avec peu de résidus visibles. On observe aussi un décollement des racines ainsi qu'un apport de volume. En outre, on observe un effet rafraîchissant immédiat, et qui perdure tout au long de la journée. L'effet parfumant perdure tout au long de la journée et est renforcé à chaque fois qu'une présence d'humidité apparait.

## Revendications

1. Composition anhydre comprenant :
a) au moins des particules encapsulant au moins un agent bénéfique, les particules étant aptes à libérer l'agent bénéfique en présence d'eau;
b) au moins une poudre absorbante de sébum présentant une prise de sébum supérieure ou égale à 35 ml/100 g et/ou au moins une poudre coiffante différente(s) des particules encapsulant au moins un agent bénéfique ;
c) un agent propulseur,
**caractérisée en ce que** *les particules encapsulant au moins un agent bénéfique comprennent au moins un matériau choisi parmi les amidons naturels ou modifiés ; et que l'agent bénéfique encapsulé est une substance parfumante.*

2. Composition selon la revendication 1, **caractérisée en ce que** la poudre absorbante de sébum est choisie parmi les amidons modifiés tels que les octénylsuccinates d'amidon et en particulier d'aluminium, la perlite, les acides polylactiques, et les zéolithes, mieux parmi les octénylsuccinates d'amidons.

3. Composition selon la revendication 1 ou 2, **caractérisée en ce que**, la ou les poudre(s) absorbante(s) de sébum est ou sont présente(s) en une quantité allant de 0,1 à 99 % en poids, de préférence de 1 à 90 % en poids, et encore plus préférentiellement de 2 à 80% en poids par rapport au poids total de la composition.

4. Composition selon l'une quelconque des revendications précédentes, **caractérisée en ce que** la poudre coiffante comprend un ou plusieurs composés minéraux insolubles dans l'eau, différents des poudres absorbantes de sébum, choisis parmi les carbonates, oxydes et sulfates métalliques et les silicates contenant du magnésium.

5. Composition selon la revendication précédente, **caractérisée en ce que** le(s) composé(s) minéral(aux) insoluble(s) dans l'eau est ou sont choisi(s) parmi le carbonate de calcium, le carbonate de magnésium, l'alumine, le sulfate de baryum, et/ou l'oxyde de magnésium, et mieux encore le carbonate de calcium.

6. Composition selon l'une quelconque des revendications 4 ou 5, **caractérisée en ce que** le(s) composé(s) minéral(aux) insoluble(s) dans l'eau est ou sont présent(s) en une quantité allant de 0,1 à 99 % en poids, mieux encore de 1 à 90 % en poids, et encore plus préférentiellement de 2 à 80 % en poids par rapport au poids total de la composition.

7. Composition selon l'une quelconque des revendications précédentes **caractérisée en ce que** les particules encapsulant au moins un agent bénéfique comprennent un cœur contenant au moins un agent bénéfique et une enveloppe entourant le cœur.

8. Composition selon l'une quelconque des revendications 1 à 6 **caractérisée en ce que** les particules encapsulant au moins un agent bénéfique comprennent une matrice poreuse, l'agent bénéfique étant contenu dans les pores de la matrice.

9. Composition selon l'une quelconque des revendications précédentes, **caractérisée en ce que** les particules encapsulant au moins un agent bénéfique sont présentes en une quantité allant de 0,01 à 10% en poids, de préférence de 0,05 à 5% en poids et encore mieux de 0,1 à 3% en poids du poids total de la composition, et en particulier lorsque le ou les agent(s) propulseur(s) sont présents dans la composition.

10. Composition selon l'une quelconque des revendications précédentes, **caractérisée en ce que** l'agent ou les agents propulseur(s) est ou sont choisi(s) parmi l'air, l'azote, le gaz carbonique, le diméthyléther, les hydrocarbures volatils, tels que notamment les alcanes en C₃₋₅, les hydrocarbures chlorés et/ou fluorés tels que le 1,1-difluoroéthane et leurs mélanges, de préférence choisis parmi les alcanes en C₃₋₅ et de préférence le n-butane, le propane, l'isobutane et leurs mélanges.

11. Composition selon l'une quelconque des revendications précédentes, **caractérisée en ce que**, lorsque le ou les agent(s) propulseur(s) sont présents dans la composition, l'agent ou les agents propulseur(s) est ou sont présent(s) en une quantité allant de 10 à 95 % en poids, mieux encore de 15 à 90 % en poids, et encore plus préférentiellement de 20 à 88 % par rapport au poids total de la composition.

12. Composition selon l'une quelconque des revendications précédentes, **caractérisée en ce qu'**elle comprend au moins un corps gras liquide.

13. Composition selon la revendication précédente, **caractérisée en ce que** le corps gras liquide est choisi parmi les hydrocarbures linéaires ou ramifiés d'origine minérale, animale ou synthétique de plus de 16 atomes de carbone, les alcools gras ramifiés ou insaturés, les esters gras, les huiles triglycérides d'origine végétale, et en particulier les huiles de paraffine ou de vaseline, l'octyldodécanol, l'alcool isostéarylique, le 2-hexyldécanol, l'alcool oléique (ou oléylique), l'alcool linoléique (ou linoléylique), l'alcool linolénique (ou linolénylique), et l'alcool undécylénique, les palmitates d'éthyle et d'isopropyle, les myristates d'alkyle tels que le myristate d'isopropyle ou d'éthyle, le stéarate d'isocétyle, l'isononanoate de 2-éthylhexyle, l'isononanoate d'isononyle, le néopentanoate d'isodécyle, et le néopentanoate d'isostéaryle, les huiles végétales, et de préférence parmi le palmitate d'isopropyle et le myristate d'isopropyle.

14. Dispositif aérosol constitué par un récipient comprenant une composition telle que définie selon l'une quelconque des revendications précédentes et par un moyen de distribution de ladite composition.

15. Procédé de lavage à sec et de traitement cosmétique des matières kératiniques, comportant une étape d'application sur les cheveux, de préférence secs, d'une composition telle que définie dans l'une quelconque des revendications 1 à 13.

16. Utilisation d'une composition selon l'une quelconque des revendications 1 à 13, pour le lavage à sec et le traitement des cheveux.

## Patentansprüche

1. Wasserfreie Zusammensetzung, umfassend:
a) mindestens Partikel, die mindestens ein vorteilhaftes Mittel einkapseln, wobei die Partikel das vorteilhafte Mittel in Gegenwart von Wasser freisetzen können;
b) mindestens ein Talg absorbierendes Pulver, das eine Talgaufnahme von mehr als oder gleich 35 ml/100 g aufweist, und/oder mindestens ein Stylingpulver, das bzw. die von den mindestens ein vorteilhaftes Mittel einkapselnden Partikeln verschieden ist bzw. sind;
c) ein Treibmittel,
**dadurch gekennzeichnet, dass** die mindestens ein vorteilhaftes Mittel einkapselnden Partikel mindestens eine aus natürlichen oder modifizierten Stärken ausgewählte Substanz umfassen und dass es sich bei dem eingekapselten vorteilhaften Mittel um einen Duftstoff handelt.

2. Zusammensetzung nach Anspruch 1, **dadurch gekennzeichnet, dass** das Talg absorbierende Pulver aus modifizierten Stärken, wie Stärkeoctenylsuccinaten und insbesondere Aluminiumstärkeoctenylsuccinaten, Perlit, Polymilchsäuren und Zeolithen, besser aus Stärkeoctenylsuccinaten, ausgewählt ist.

3. Zusammensetzung nach Anspruch 1 oder 2, **dadurch gekennzeichnet, dass** das oder die Talg absorbierende(n) Pulver in einer Menge von 0,1 bis 99 Gew.-%, vorzugsweise von 1 bis 90 Gew.-% und noch stärker bevorzugt von 2 bis 80 Gew.-%, bezogen auf das Gesamtgewicht der Zusammensetzung, vorliegt bzw. vorliegen.

4. Zusammensetzung nach einem der vorhergehenden Ansprüche, **dadurch gekennzeichnet, dass** das Stylingpulver eine oder mehrere wasserunlösliche Mineralverbindungen, die von den Talg absorbierenden Pulvern verschieden sind, ausgewählt aus Metallcarbonaten, -oxiden und -sulfaten und Magnesium enthaltenden Silikaten, umfasst.

5. Zusammensetzung nach dem vorhergehenden Anspruch, **dadurch gekennzeichnet, dass** die wasserunlösliche(n) Mineralverbindung(en) aus Calciumcarbonat, Magnesiumcarbonat, Aluminiumoxid, Bariumsulfat und/oder Magnesiumoxid und noch besser aus Calciumcarbonat ausgewählt ist bzw. sind.

6. Zusammensetzung nach einem der Ansprüche 4 oder 5, **dadurch gekennzeichnet, dass** die wasserunlösliche(n) Mineralverbindung(en) in einer Menge von 0,1 bis 99 Gew.-%, noch besser von 1 bis 90 Gew.-% und noch stärker bevorzugt von 2 bis 80 Gew.-%, bezogen auf das Gesamtgewicht der Zusammensetzung, vorliegt bzw. vorliegen.

7. Zusammensetzung nach einem der vorhergehenden Ansprüche, **dadurch gekennzeichnet, dass** die mindestens ein vorteilhaftes Mittel einkapselnden Partikel einen Kern, der das mindestens ein vorteilhaftes Mittel enthält, und eine den Kern umgebende Hülle umfassen.

8. Zusammensetzung nach einem der Ansprüche 1 bis 6, **dadurch gekennzeichnet, dass** die mindestens ein vorteilhaftes Mittel einkapselnden Partikel eine poröse Matrix umfassen, wobei das vorteilhafte Mittel in den Poren der Matrix enthalten ist.

9. Zusammensetzung nach einem der vorhergehenden Ansprüche, **dadurch gekennzeichnet, dass** die mindestens ein vorteilhaftes Mittel einkapselnden Partikel in einer Menge von 0,01 bis 10 Gew.-%, vorzugsweise von 0,05 bis 5 Gew.-% und noch besser von 0,1 bis 3 Gew.-%, bezogen auf das Gesamtgewicht der Zusammensetzung, und insbesondere wenn das oder die Treibmittel in der Zusammensetzung vorhanden ist bzw. sind, vorliegen.

10. Zusammensetzung nach einem der vorhergehenden Ansprüche, **dadurch gekennzeichnet, dass** das oder die Treibmittel aus Luft, Stickstoff, Kohlendioxid, Dimethylether, flüchtigen Kohlenwasserstoffen, wie insbesondere C₃₋₅Alkanen, chlorierten und/oder fluorierten Kohlenwasserstoffen, wie 1,1-Difluorethan, und ihren Gemischen ausgewählt, vorzugsweise aus C₃₋₅Alkanen und vorzugsweise n-Butan, Propan, Isobutan und ihren Gemischen ausgewählt ist bzw. sind.

11. Zusammensetzung nach einem der vorhergehenden Ansprüche, **dadurch gekennzeichnet, dass**, wenn das oder die Treibmittel in der Zusammensetzung vorhanden ist bzw. sind, das oder die Treibmittel in der Zusammensetzung in einer Menge von 10 bis 95 Gew.-%, noch besser von 15 bis 90 Gew.-% und noch stärker bevorzugt von 20 bis 88 Gew.-%, bezogen auf das Gesamtgewicht der Zusammensetzung, vorliegt bzw. vorliegen.

12. Zusammensetzung nach einem der vorhergehenden Ansprüche, **dadurch gekennzeichnet, dass** sie mindestens eine flüssige Fettsubstanz umfasst.

13. Zusammensetzung nach dem vorhergehenden Anspruch, **dadurch gekennzeichnet, dass** die flüssige Fettsubstanz aus geraden oder verzweigten Kohlenwasserstoffen anorganischen, tierischen oder synthetischen Ursprungs mit mehr als 16 Kohlenstoffatomen, verzweigten oder ungesättigten Fettalkoholen, Fettestern, Triglyceridölen pflanzlichen Ursprungs und insbesondere Paraffin- oder Vaselineölen, Octyldodecanol, Isostearylalkohol, 2-Hexyldecanol, Oleylalkohol, Linoleylalkohol, Linolenylalkohol und Undecylenylalkohol, Ethyl- und Isopropylpalmitat, Alkylmyristaten, wie Isopropyl- oder Ethylmyristat, Isocetylstearat, 2-Ethylhexylisononanoat, Isononylisononanoat, Isodecylneopentanoat und Isostearylneopentanoat, Pflanzenölen und vorzugsweise aus Isopropylpalmitat und Isopropylmyristat ausgewählt ist.

14. Aerosolvorrichtung, bestehend aus einem Behälter, der eine Zusammensetzung nach einem der vorhergehenden Ansprüche umfasst, und einem Mittel zum Abgeben der Zusammensetzung.

15. Verfahren zur Trockenwäsche und zur kosmetischen Behandlung von Keratinsubstanzen, das einen Schritt des Aufbringens einer Zusammensetzung nach einem der Ansprüche 1 bis 13 auf die, vorzugsweise trockenen, Haare umfasst.

16. Verwendung einer Zusammensetzung nach einem der Ansprüche 1 bis 13 zur Trockenwäsche und zur Behandlung der Haare.

## Claims

1. Anhydrous composition comprising:
a) at least particles encapsulating at least one beneficial agent, the particles being able to release the beneficial agent in the presence of water;
b) at least one sebum-absorbing powder with a sebum uptake of greater than or equal to 35 ml/100 g, and/or at least one styling powder other than the particles encapsulating at least one beneficial agent;
c) a propellant,
**characterized in that** *the particles encapsulating at least one beneficial agent comprise at least one material chosen from natural or modified starches; and that the encapsulated beneficial agent is a fragrancing substance.*

2. Composition according to Claim 1, **characterized in that** the sebum-absorbing powder is chosen from modified starches such as starch octenylsuccinates and in particular aluminium starch octenylsuccinates, perlite, polylactic acids and zeolites, and better still from starch octenylsuccinates.

3. Composition according to Claim 1 or 2, **characterized in that** the sebum-absorbing powder(s) is or are present in an amount ranging from 0.1% to 99% by weight, preferably from 1% to 90% by weight and even more preferentially from 2% to 80% by weight, relative to the total weight of the composition.

4. Composition according to any one of the preceding claims, **characterized in that** the styling powder comprises one or more water-insoluble mineral compounds other than the sebum-absorbing powders, chosen from metal carbonates, oxides and sulfates, and silicates containing magnesium.

5. Composition according to the preceding claim, **characterized in that** the water-insoluble mineral compound(s) is or are chosen from calcium carbonate, magnesium carbonate, alumina, barium sulfate and/or magnesium oxide, and even better still calcium carbonate.

6. Composition according to either one of Claims 4 and 5, **characterized in that** the water-insoluble mineral compound(s) is or are present in an amount ranging from 0.1% to 99% by weight, even better still from 1% to 90% by weight and even more preferentially from 2% to 80% by weight, relative to the total weight of the composition.

7. Composition according to any one of the preceding claims, **characterized in that** the particles encapsulating at least one beneficial agent comprise a core containing at least one beneficial agent and a shell surrounding the core.

8. Composition according to any one of Claims 1 to 6, **characterized in that** the particles encapsulating at least one beneficial agent comprise a porous matrix, the beneficial agent being contained in the pores of the matrix.

9. Composition according to any one of the preceding claims, **characterized in that** the particles encapsulating at least one beneficial agent are present in an amount ranging from 0.01% to 10% by weight, preferably from 0.05% to 5% by weight and better still from 0.1% to 3% by weight of the total weight of the composition, and in particular when the propellant(s) are present in the composition.

10. Composition according to any one of the preceding claims, **characterized in that** the propellant (s) is or are chosen from air, nitrogen, carbon dioxide, dimethyl ether, volatile hydrocarbons such as, in particular, C₃₋₅ alkanes, chlorinated and/or fluorinated hydrocarbons such as 1,1-difluoroethane and mixtures thereof, preferably chosen from C₃₋₅ alkanes and preferably n-butane, propane, isobutane and mixtures thereof.

11. Composition according to any one of the preceding claims, **characterized in that**, when the propellant(s) is or are present in the composition, the propellant(s) is or are present in an amount ranging from 10% to 95% by weight, even better still from 15% to 90% by weight, and even more preferably from 20% to 88% by weight, relative to the total weight of the composition.

12. Composition according to any of the preceding claims, **characterized in that** it comprises at least one liquid fatty substance.

13. Composition according to the preceding claim, **characterized in that** the liquid fatty substance is chosen from linear or branched hydrocarbons of mineral, animal or synthetic origin and with more than 16 carbon atoms, branched or unsaturated fatty alcohols, fatty esters, triglyceride oils of plant origin, and in particular liquid paraffin or liquid petroleum jelly, octyldodecanol, isostearyl alcohol, 2-hexyldecanol, oleyl alcohol, linoleyl alcohol, linolenyl alcohol and undecylenyl alcohol, ethyl and isopropyl palmitates, alkyl myristates such as isopropyl myristate or ethyl myristate, isocetyl stearate, 2-ethylhexyl isononanoate, isononyl isononanoate, isodecyl neopentanoate and isostearyl neopentanoate, and plant oils, and preferably from isopropyl palmitate and isopropyl myristate.

14. Aerosol device constituted of a container comprising a composition as defined according to any one of the preceding claims and of a means for dispensing said composition.

15. Process for the dry-washing and cosmetic treatment of keratin materials, comprising a step of applying to the hair, preferably dry hair, a composition as defined in any one of Claims 1 to 13.

16. Use of a composition according to any one of Claims 1 to 13, for the dry-washing and treatment of the hair.
